(19) Europäisches Patentamt
European Patent Office
Office européen des brevets

(11) Publication number: **0 015 702**
**B1**

(12) # EUROPEAN PATENT SPECIFICATION

(45) Date of publication of patent specification: **19.05.82**

(51) Int. Cl.³: **C 01 B 33/28, B 01 J 29/28**

(21) Application number: **80300561.0**

(22) Date of filing: **26.02.80**

(54) **Crystalline zeolite, synthesis and use thereof.**

(30) Priority: **28.02.79 US 16248**

(43) Date of publication of application: **17.09.80 Bulletin 80/19**

(45) Publication of the grant of the patent: **19.05.82 Bulletin 82/20**

(84) Designated Contracting States: **BE DE FR GB IT NL**

(56) References cited:
**EP - A - 0 001 695**
**DE - A - 2 748 278**
**DE - A - 2 749 024**
**US - A - 4 046 859**

(73) Proprietor: **MOBIL OIL CORPORATION**
**150 East 42nd Street**
**New York New York 10017 (US)**

(72) Inventor: **Doherty, Harry George**
**210 Adams Avenue**
**Pitman, New Jersey 08071 (US)**
Inventor: **Rosinski, Edward Joseph**
**Route 1, Box 325a**
**Pedricktown, New Jersey 08067 (US)**
Inventor: **Plank, Charles Joseph**
**522 Delaware Street**
**Woodbury, New Jersey 08096 (US)**

(74) Representative: **Cooper, John Anthony et al,**
**Mobil Court 3 Clements Inn**
**London WC2A 2EB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 015 702 B1

# Crystalline zeolite, synthesis and use thereof

This invention relates to a new crystalline zeolite, to a method for synthesising it, and to its use, inter alia as hydrocarbon conversion catalyst.

Many crystalline zeolites are known, in most cases as aluminosilicates. Some occur (at least so far) only in nature, for instance paulingite and merlinoite; some occur only as a result of synthesis, for instance zeolites A and ZSM-5; and some occur in both natural and synthetic forms, for instance mordenite, a synthetic counterpart of which is known as Zeolon, and faujasite, synthetic counterparts of which are known as zeolites X and Y. Counterparts are of course demonstrated as such by correspondence of their X-ray diffraction data, the indicia by means of which the individuality of a zeolite is established. Such data are a manifestation of the particular geometry of the three-dimensional lattice, formed of $SiO_4$ and in most cases $AlO_4$ tetrahedra crosslinked by the sharing of oxygen atoms and including sufficient cationic complement to balance the resulting negative charge on any $AlO_4$ tetrahedra, of which a zeolite consists.

We have now discovered a zeolite having a lattice structure previously unknown, as evidenced by its X-ray diffraction data, which we call ZSM-25.

According to the present invention a crystalline aluminosilicate zeolite, ZSM-25, has a lattice constituted by $SiO_4$ and $AlO_4$ tetrahedra crosslinked by the sharing of oxygen atoms and characterized by the following interplanar spacings:

Table I

| 2θ | d(A°) | Relative Intensity | 2θ | d(A°) | Relative Intensity |
|---|---|---|---|---|---|
| 9.94 | 8.90±.18 | W | 22.60 | 3.93±.08 | W |
| 11.00 | 8.04±.16 | VS | 24.12 | 3.69±.08 | S |
| 12.05 | 7.34±.15 | W | 26.61 | 3.35±.07 | M |
| 12.63 | 7.01±.14 | VS | 26.89 | 3.32±.07 | VS |
| 13.79 | 6.42±.13 | S | 27.47 | 3.25±.07 | VS |
| 14.62 | 6.06±.12 | M | 28.33 | 3.15±.06 | M |
| 15.40 | 5.75±.12 | M | 28.74 | 3.11±.06 | VS |
| 15.70 | 5.64±.12 | M | 29.44 | 3.03±.06 | S |
| 16.83 | 5.27±.11 | M | 30.74 | 2.91±.06 | W |
| 17.31 | 5.12±.11 | W | 31.85 | 2.81±.06 | M |
| 17.73 | 5.00±.10 | M | 32.93 | 2.72±.06 | M |
| 19.24 | 4.61±.09 | VS | 34.00 | 2.64±.05 | M |
| 19.62 | 4.52±.09 | M | 40.07 | 2.25±.05 | W |
| 21.72 | 4.09±.08 | M | 44.24 | 2.05±.04 | W |

These values were determined by standard technique. The radiation was the K-alpha doublet of copper, and a scintillation counter spectrometer with a strip chart pen recorder was used. The positions as a function of 2 times theta, where theta is the Bragg angle, were read from the spectrometer chart, and values for d (A°), the interplanar spacing in Angstrom units, corresponding to the recorded lines, were calculated. This X-ray diffraction pattern is characteristic of all cation forms of ZSM-25, although, as is well understood in the art, minor shifts in interplanar spacing can occur with change of cation, variation of silicon to aluminum ratio, and thermal treatment. In the Table W=week, VS=very strong, S=strong and M=medium. Corresponding relative intensities expressed numerically with reference to the highest peak are: for VS, 60—100; for S, 40—60; for M, 20—40; and for W, 0—20.

Zeolite ZSM-25 possesses the formula

$$M_{2/n}O:Al_2O_3:6\text{—}10\ SiO_2$$

where M is one or more cations of valence n. In the anhydrous as-synthesised form the zeolite usually manifests the formula:

$$(0.01\text{—}0.4)R_2O:(0.9\pm0.2)\ M_2O:Al_2O_3:(6\text{—}10)\ SiO_2$$

wherein R is an organic nitrogen-containing cation containing at least one alkyl or aryl group having between 1 and 7 carbon atoms, preferably between 2 and 5 carbon atoms, more preferably containing at least one ethyl group, and still more preferably R is a quarternary ammonium cation containing at least one ethyl group, particularly tetraethylammonium, and M is an alkali metal cation, especially sodium.

The original cations can be replaced in accordance with techniques well known at least in part by

ion exchange with other cations. Preferred replacing cations include metal ions, ammonium ions, and mixtures of the same. Particularly preferred cations are those which render the zeolite catalytically active, especially for hydrocarbon conversion. These include hydrogen, calcium magnesium, zinc, rare earth metals, aluminum, metals of Groups II and VIII of the Periodic Table, nickel and manganese. Pore blockage by organic cations or residues may require clearance by calcination before ion exchange is attempted, suitably in an atmosphere of air steam, nitrogen, hydrogen or oxygen at a temperature of 400 to 900°C.

Zeolite ZSM-25 is useful as an absorbent and is catalytically active in a broad area of hydrocarbon conversion reactions such as polymerization, oligomerization, isomerization and hydrocracking. ZSM-25 is thermally stable under the conditions encountered in catalytic cracking and catalyst regeneration.

ZSM-25 can be used either in the alkali metal form, e.g. the sodium form, the ammonium form, the hydrogen form, or another univalent or multivalent cationic form. The zeolite can also be used in intimate combination with a hydrogenating component such as tungsten, vanadium, molybdenum, rhenium, nickel, cobalt, chromium, magnesium, or a noble metal, such as platinum or palladium where a hydrogenation dehydrogenation function is to be performed. Such components can be exchanged into the zeolite impregnated therein or physically intimately admixed therewith. In the case of platinum, impregnation can be effected by treating the zeolite with a platinum metal-containing ion such as chloroplatinic acid, platinuous chloride and various compounds containing the platinum amine complex. Combinations of metals and methods for their introduction can also be used. The metal may be present in the anion or cation of the compound, the cationic complex, e.g., $Pt(NH_3)_6Cl_4$, being particularly useful. For some hydrocarbon conversion processes ZSM-25 requires no noble metal component such as in low temperature, liquid phase orthoxylene isomerization.

In the synthesis of ZSM-25 an organic nitrogen compound containing a cation such as tetraethylammonium is employed, a preferred compound being tetraethylammonium bromide, although the chloride sulphate and hydroxide may also be advantageously used. A variety of alkali metal cations may be used suitably defined as including all alkali metal ions derived from alkali metal oxide or hydroxide as well as alkali metal ions included in alkali metal silicates and aluminates (not including alkali metal salts such as sodium chloride or sodium sulfate which may be derived from neutralization of added inorganic acids such as HCl or $H_2SO_4$ or acid salts such as $Al_2(SO_4)_3$). Non-limiting examples of such suitable alkali metal ions include sodium and potassium.

When employed either as an adsorbent or as a catalyst the zeolite should be dehydrated at least partially, suitably by heating to a temperature in the range of 200° to 600°C in an atmosphere such as air, nitrogen, steam, etc. and at atmospheric or subatmospheric pressures for a time between 1 and 48 hours. Dehydration can also be performed at lower temperature merely by placing a catalyst in a vacuum but a longer time is required to obtain sufficient dehydration.

Zeolite ZSM-25 can be synthesised by preparing a solution containing sources of tetraethylammonium cations, sodium oxide, an oxide of aluminum, an oxide of silicon, and water having a composition in terms of mole ratios of oxides falling within the following ranges:

| | | Preferred |
|---|---|---|
| $SiO_2/Al_2O_3$ | 5—15 | 8—10 |
| $R^+/R^++M^+$ | 0.4—1.0 | 0.6—0.8 |
| $OH^-/SiO_2$ | 0.3—0.7 | 0.4—0.6 |
| $H_2O/OH^-$ | 50—300 | 60—150 |

(where R and M are as defined above) and maintaining the mixture until crystals of the zeolite are formed. Thereafter, the crystals are separated from the liquid and recovered. Typical reaction conditions consist of heating the foregoing reaction mixture to a temperature of 80°C to 180°C at a pressure within the range 618.5 kPa (75 psig) to 1480.3 kPa (200 psig) for six hours to 150 days. A more preferred temperature range is from 121°C to 149°C at a pressure within the range of 790.8 kPa (100) to 1307.9 kPa (175 psig) for about four days.

In a specific, preferred procedure ZSM-25 is synthesized from a mixture containing colloidal silica, sodium aluminate, sodium hydroxide, tetraethylammonium compounds, and water at a crystallization temperature of 135°C, at a pressure of 790.8—1307.9 kPa (100—175 psig). The product is dried at 110°C for about 16 to 24 hours. Milder conditions may be employed for the drying if desired, such as at room temperature under vacuum.

A variety of materials can be employed to supply the appropriate oxide in the reaction mixture. Such compositions include for an aluminosilicate, sodium aluminate, colloidal silica, sodium hydroxide, and tetraethylammonium compounds such as tetraethylammonium bromide. Each oxide component utilized in the reaction mixture for preparing ZSM-25 can be supplied by one or more initial reactants and they can be mixed together in any order. For example, sodium oxide can be prepared by an aqueous solution of sodium hydroxide or by an aqueous solution of sodium silicate; tetraethylammonium cation can be supplied by tetraethylammonium hydroxide, tetraethylammonium bromide, or tetraethylammonium chloride or tetraethylammonium sulfate. The reaction mixture can be prepared either

batchwise or continuously. Crystal size and crystallization time will vary with the nature of the reaction mixture employed.

ZSM-25 may be formed in a wide variety of particle sizes. The particles can be in the form of a powder, a granule, or molded product, such as extrudate having particle size sufficient to pass through a 2-mesh (Tyler) screen and be retained on a 400-mesh (Tyler) screen. In cases where a catalyst is molded, such as by extrusion, the aluminosilicate can be extruded before drying or dried or partially dried and then extruded. As in the case of many catalysts, it can be desirable to incorporate ZSM-25 in a matrix material resistant to the temperatures and other conditions employed in organic conversion processes, as described in our European Specification 0,001,695.

Employing a ZSM-25 catalyst containing a hydrogenation component, heavy petroleum residual stocks, cycles stocks and other hydrocrackable charge stocks can be hydrocracked at temperatures between 204.5°C (400°F) and 440.6°C (825°F) using molar ratios of hydrogen to hydrocarbon charge in the range of 2 to 80. The pressure employed will vary between 170.3 kPa (10 psig) and 17338.3 kPa (2,500 psig) and the liquid hourly space velocity between 0.1 and 10.

Employing the zeolite for catalytic cracking, hydrocarbon cracking stocks can be cracked at a liquid hourly space velocity between about 0.5 and 50, a temperature between about 287.8°C (550°F) and 593.4°C (1100°F), a pressure between about subatmospheric and several hundred atmospheres.

Employing a catalytically active form of ZSM-25 containing a hydrogenation component, reforming stocks can be reformed employing a temperature between 371.2°C (700°F) and 537.8°C (1000°F). The pressure can be between 790.8 kPa (100 psig) and 6996.1 kPa (1000 psig) but is preferably between 1480.3 kPa (200 psig) and 4927.7 kPa (700 psig). The liquid hourly space velocity is generally between 0.1 and 10, preferably between 0.5 and 4 and the hydrogen to hydrocarbon mole ratio is generally between 1 and 20, preferably between 4 and 12.

The zeolite can be used for hydroisomerization of normal paraffins, when provided with a hydrogenation component, e.g., platinum. Hydroisomerization is carried out at a temperature between 93.4°C (200°F) and 371.2°C (700°F), preferably 148.9°C (300°F) and 287.8°C (550°F) and with a liquid hourly space velocity between 0.01 and 2, preferably between 0.25 and 0.50 employing hydrogen such that the hydrogen to hydrocarbon mole ratio is between 1:1 and 5:1. Additionally, the catalyst can be used for olefin and aromatic isomerization employing temperatures between —1.2°C (30°F) and 482.3°C (900°F), preferably 65.6°C (150°F) to about 315.6°C (600°F).

The zeolite can also be used for the oligomerization (polymerization) of olefins at a temperature of about 260°C (500°F) to about 482.3°C (900°F) and a liquid hourly space velocity of about 0.1 to about 10: and for reducing the pour point of gas oils, at a liquid hourly space velocity between about 1 and about 30 and at a temperature between about 426.7°C (800°F) and about 593.4°C (1100°F). Other reactions which can be catalysed by the zerolite containing a metal, e.g., platinum, include hydrogenation-dehydrogenation and desulfurization.

In the examples which follow by way of illustration of the invention, adsorption data was determined as follows:

A weighed sample of the zeolite was contacted with the pure adsorbate vapor in an adsorption chamber at a pressure less than the vapor-liquid equilibrium pressure of the adsorbate at room temperature. This pressure was kept constant during the adsorption period, which did not exceed about eight hours. Adsorption was complete when a constant pressure in the adsorption chamber was reached, i.e., 12 mm of mercury for water and 20 mm for n-hexane and cyclohexane. The increase in weight was calculated as the adsorption capacity of the same.

Example 1

Zeolite ZSM-25 was synthesized from a mixture formed from 228.0 grams colloidal silica (30% $SiO_2$), 30.0 grams $NaAlO_2$, 8.7 grams sodium hydroxide (NaOH), 330.0 grams tetraethylammonium bromide ($(CH_3CH_2)_4NBr$) and 282 grams water ($H_2O$), and having the following molar composition expressed in terms of mole ratios:

| | |
|---|---|
| $SiO_2/Al_2O_3$ | 9.0 |
| $R^+/R^++M^+$ | 0.75 |
| $OH^-/SiO_2$ | 0.47 |
| $H_2O/OH^-$ | 101 |

The pH of the mixtures ranges from about 13.7 to 14.7.

Crystallization was carried out in a glass-lined stirring autoclave at 135°C (275°F) and a pressure of 790.9—1307.9 kPa (100—175 psig). The time for crystallization was four days. The resulting solid product was cooled to room temperature, removed, filtered, washed with water and dried at 110°C (230°F).

Chemical analysis of the product dried at 110°C (230°F) showed the formula to be:

$$(0.12)\ R_2O:(0.87)\ Na_2O:Al_2O_3:(8.5)\ SiO_2$$

where R is a tetraethylammonium ion. The product contained 3.8% by weight carbon and 0.5% by weight nitrogen.

A portion of the un-calcined product was subjected to X-ray analysis and identified as ZSM-25. The material exhibited the X-ray powder diffraction pattern as shown in Fig. 1 and Table II.

Table II
X-ray data ZSM-25 noncalcined

| $2\theta$ | d | Relative Intensity | $2\theta$ | d | Relative Intensity |
|---|---|---|---|---|---|
| 5.00 | 16.06 | 2 | 33.61 | 2.666 | 9 |
| 7.20 | 12.28 | 2 | 33.98 | 2.638 | 40 |
| 7.60 | 11.63 | 2 | 34.46 | 2.603 | 4 |
| 8.23 | 10.74 | 2 | 35.10 | 2.557 | 4 |
| 9.56 | 9.25 | 4 | 35.82 | 2.507 | 10 |
| 9.90 | 8.93 | 7 | 36.13 | 2.486 | 3 |
| 10.95 | 8.08 | 67 | 37.55 | 2.395 | 2 |
| 12.00 | 7.37 | 13 | 37.90 | 2.374 | 4 |
| 12.58 | 7.04 | 86 | 38.33 | 2.348 | 3 |
| 13.74 | 6.44 | 56 | 38.67 | 2.328 | 2 |
| 14.25 | 6.22 | 5 | 39.04 | 2.307 | 2 |
| 14.58 | 6.08 | 25 | 39.47 | 2.283 | 3 |
| 15.39 | 5.76 | 20 | 40.03 | 2.252 | 7 |
| 15.61 | 5.68 | 20 | 40.87 | 2.208 | 2 |
| 16.13 | 5.49 | 2 | 41.16 | 2.193 | 2 |
| 16.80 | 5.28 | 30 | 41.56 | 2.173 | 1 |
| 17.27 | 5.13 | 14 | 41.91 | 2.156 | 2 |
| 17.70 | 5.01 | 34 | 42.22 | 2.140 | 4 |
| 18.51 | 4.79 | 6 | 42.70 | 2.118 | 1 |
| 19.19 | 4.62 | 72 | 43.40 | 2.085 | 1 |
| 19.56 | 4.54 | 26 | 43.62 | 2.075 | 1 |
| 19.99 | 4.44 | 6 | 44.19 | 2.050 | 12 |
| 20.55 | 4.32 | 3 | 44.56 | 2.033 | 3 |
| 20.93 | 4.24 | 12 | 45.08 | 2.011 | 2 |
| 21.66 | 4.10 | 29 | 45.32 | 2.001 | 5 |
| 22.34 | 3.98 | 8 | 46.20 | 1.965 | 3 |
| 22.54 | 3.94 | 15 | 46.48 | 1.954 | 4 |
| 23.77 | 3.74 | 11 | 46.68 | 1.946 | 4 |
| 24.07 | 3.70 | 44 | 47.05 | 1.931 | 2 |
| 24.85 | 3.58 | 8 | 47.83 | 1.902 | 3 |
| 25.15 | 3.54 | 9 | 48.63 | 1.872 | 1 |
| 26.56 | 3.36 | 22 | 49.17 | 1.853 | 2 |
| 26.83 | 3.32 | 67 | 50.63 | 1.803 | 1 |
| 27.42 | 3.25 | 100 | 51.50 | 1.774 | 35 |
| 28.30 | 3.15 | 34 | 52.08 | 1.756 | 3 |
| 28.68 | 3.11 | 80 | 52.68 | 1.737 | 7 |
| 29.38 | 3.04 | 50 | 54.15 | 1.694 | 5 |
| 29.97 | 2.981 | 3 | 54.38 | 1.687 | 6 |
| 30.35 | 2.945 | 8 | 54.85 | 1.674 | 6 |
| 30.74 | 2.909 | 12 | 55.69 | 1.650 | 2 |
| 31.52 | 2.838 | 11 | 56.56 | 1.627 | 5 |
| 31.80 | 2.814 | 22 | 57.11 | 1.613 | 3 |
| 32.30 | 2.772 | 13 | 58.10 | 1.588 | 5 |
| 32.87 | 2.725 | 28 | 58.98 | 1.566 | 3 |
| | | | 59.80 | 1.547 | <1 |

A portion of the product was calcined at 537.8°C (1000°F) in air for five hours and furnished the following analysis:

| | |
|---|---|
| Wt. percent $SiO_2$ | 77.3 |
| Wt. percent $Al_2O_3$ | 15.5 |
| Wt. percent Na | 6.1 |
| $SiO_2/Al_2O_3$ | 8.5 |

and the following adsorption data:

| | |
|---|---|
| Wt. percent n-hexane adsorbed | 10.87 |
| Wt. percent cyclohexane adsorbed | 3.60 |
| Wt. percent $H_2O$ adsorbed | 9.15 |

Example II

A sample of the product of Example I was calcined for four hours at 500°C in a muffle furnace. Some loss in diffracted intensity and some broadening of lines indicated some lattice distortion with some loss in crystallinity. The X-ray diffraction pattern of the calcined sample is shown in Fig. 2 and Table III.

Table III
X-ray data ZSM-25 calcined 4 hrs at 500°C

| 2θ | d | Relative Intensity | 2θ | d | Relative Intensity |
|---|---|---|---|---|---|
| 7.18 | 12.03 | 2 | 27.50 | 3.23 | 100 |
| 7.50 | 11.08 | 2 | 28.30 | 3.15 | 22 |
| 8.20 | 10.08 | 2 | 28.80 | 3.10 | 85 |
| 9.50 | 9.31 | 6 | 29.50 | 3.03 | 44 |
| 9.90 | 8.93 | 13 | 29.90 | 2.99 | 6 |
| 10.98 | 8.06 | 96 | 30.40 | 2.94 | 6 |
| 11.95 | 7.41 | 13 | 30.82 | 2.90 | 6 |
| 12.60 | 7.03 | 87 | 31.60 | 2.83 | 6 |
| 13.78 | 6.43 | 80 | 31.90 | 2.80 | 20 |
| 14.20 | 6.24 | 11 | 32.30 | 2.77 | 13 |
| 14.60 | 6.07 | 22 | 32.90 | 2.72 | 20 |
| 15.38 | 5.76 | 22 | 33.70 | 2.66 | 9 |
| 15.70 | 5.64 | 24 | 34.10 | 2.63 | 28 |
| 16.20 | 5.47 | 6 | 34.60 | 2.59 | 4 |
| 16.88 | 5.25 | 22 | 35.22 | 2.55 | 2 |
| 17.35 | 5.11 | 11 | 35.90 | 2.50 | 6 |
| 17.78 | 4.99 | 35 | 36.20 | 2.48 | 4 |
| 18.60 | 4.77 | 6 | 37.90 | 2.37 | 3 |
| 19.25 | 4.61 | 59 | 38.30 | 2.35 | 5 |
| 19.70 | 4.51 | 21 | 38.80 | 2.32 | 3 |
| 20.10 | 4.42 | 6 | 40.10 | 2.25 | 3 |
| 20.50 | 4.33 | 4 | 42.60 | 2.12 | 3 |
| 20.97 | 4.24 | 11 | 43.00 | 2.10 | 2 |
| 21.75 | 4.09 | 22 | 44.25 | 2.05 | 6 |
| 22.17 | 4.01 | 7 | 44.58 | 2.03 | 6 |
| 22.62 | 3.93 | 13 | 45.40 | 1.998 | 4 |
| 23.60 | 3.77 | 7 | 46.10 | 1.969 | 4 |
| 24.15 | 3.69 | 43 | 46.70 | 1.945 | 6 |
| 24.85 | 3.58 | 9 | 47.20 | 1.926 | 4 |
| 25.25 | 3.53 | 9 | 51.70 | 1.768 | 24 |
| 26.50 | 3.36 | 17 | 52.30 | 1.749 | 9 |
| 26.90 | 3.31 | 68 | 52.90 | 1.731 | 6 |

Example III

A further sample of the zeolite was subjected to ammonium chloride exchange reducing the Na level to 0.2 weight percent from an initial value of 6.1 weight percent. A sample of the exchanged zeolite was evaluated for hydrocarbon cracking activity in the n-hexane cracking test (alpha test), and showed a cracking activity about 23 times greater than a standard silica-alumina catalyst.

Example IV

In an oligomerization test run to illustrate the activity of the exchanged zeolite for hydrocarbon conversion 371.2°C (700°F) the sample converted 11.6 weight percent propylene to propylene oligomers.

The alpha test was carried out as follows. n-hexane diluted with helium was passed over a 1.0 cc sample of the c atalyst at a liquid hourly space velocity=1 and at 482.3°C (900°F). The conversion of hexane to cracked products at 5 minutes on stream was equal to 22.7%. This calculates to a relative cracking activity ($\alpha$ value) of 22.9.

The oligomerization test was carried out as follows: Propylene was charged at the rate of 16.66 cc/min (1000 cc/hr) over a 0.259 g (0.67 cc) sample of the catalyst at 371.2°C (700°F) for a two-hour period. A liquid balance was made during the second hour on stream. On the basis of recovered hydrocarbons 14.4% of the propylene was converted. Of this, 83.0% was converted to $C_4$+ and 57.1% $C_5$+ hydrocarbons.

## Claims

1. A crystalline aluminosilicate zeolite having a lattice constituted by $SiO_4$ and $AlO_4$ tetrahedra crosslinked by the sharing of oxygen atoms and characterized by the following interplanar spacings and relative intensities of its X-ray diffraction bands:

| d (A°) | Relative Intensity | d (A°) | Relative Intensity |
|---|---|---|---|
| 8.90±.18 | W | 3.93±.08 | W |
| 8.04±.16 | VS | 3.69±.08 | S |
| 7.34±.15 | W | 3.35±.07 | M |
| 7.01±.14 | VS | 3.32±.07 | VS |
| 6.42±.13 | S | 3.25±.07 | VS |
| 6.06±.12 | M | 3.15±.06 | M |
| 5 75±.12 | M | 3.11±.06 | VS |
| 5.64±.12 | M | 3.03±.06 | S |
| 5.27±.11 | M | 2.91±.06 | W |
| 5.12±.11 | W | 2.81±.06 | M |
| 5.00±.10 | M | 2.72±.06 | M |
| 4.61±.09 | VS | 2.64±.05 | M |
| 4.52±.09 | M | 2.25±.05 | W |
| 4.09±.08 | M | 2.05±.04 | W |

said zeolite having the formula

$$M_{2/n}O:Al_2O_3:6—10\ SiO_2$$

where M is one or more cations of valence n.

2. A zeolite according to Claim 1 wherein M represents alkali metal and a nitrogen-containing organic cation.

3. A zeolite according to Claim 1 or Claim 2 which in the anhydrous form has the formula:

$$(0.01—0.4)\ R_2O:(0.9\pm0.2)\ M_2O:Al_2O_3:(6—10)\ SiO_2$$

in which M is alkali metal and R is nitrogen-containing organic cation.

4. A zeolite according to Claim 2 or Claim 3 wherein the nitrogen-containing cation contains at least one alkyl or aryl group having from 1 to 7 carbon atoms.

5. A zeolite according to any of Claims 2 to 4 wherein the nitrogen-containing organic cation is tetraethylammonium.

6. A zeolite according to any of Claims 1 to 5 the original cations of which have been at least partly removed by thermal treatment and/or base exchange.

7. A zeolite according to Claim 6 which contains hydrogen, hydrogen precursor and/or rare earth cations.

8. A method of preparing the zeolite claimed in Claim 1 which comprises preparing a mixture containing silica, sodium aluminate, sodium hydroxide, tetraethylammonium bromide and water and having a composition in terms of mole ratios of oxides:

| | |
|---|---|
| $SiO_2/Al_2O_3$ | 5.0—15 |
| $R^+/R^++M^+$ | 0.4—1.0 |
| $OH^-/SiO_2$ | 0.3—0.7 |
| $H_2O/OH^-$ | 50—300 |

(wherein $R^+$ is a tetraethylammonium cation and $M^+$ is an alkali metal cation), maintaining said mixture at a temperature of 80°C to 180°C and a pressure of 618.5 kPa (75 psig) to 1480.3 kPa (200 psig) until crystals of the zeolite form, and separating and recovering said crystals.

9. A method according to Claim 8 wherein said mixture has the composition:

| | |
|---|---|
| $SiO_2/Al_2O_3$ | 8.0—10.0 |
| $R^+/R^++M^+$ | 0.6—0.8 |
| $OH^-/SiO_2$ | 0.4—0.6 |
| $H_2O/OH^-$ | 60—150 |

and is maintained at a temperature within the range 121 °C to 149°C and a pressure within the range 790.8 kPa (100 psig) to 1307.9 kPa (175 psig).

10. A method according to Claim 8 or Claim 9 wherein $R^+$ is derived from tetraethylammonium bromide and wherein $M^+$ is sodium.

11. A method according to any of Claims 8 to 10 wherein said mixture is maintained at a temperature of about 135°C for a time of about 96 hours.

12. Use of the crystalline aluminosilicate zeolite as claimed in any of Claims 1 to 7 as a catalyst in the conversion of hydrocarbons.

**Revendications**

1. Zéolite d'aluminosilicate cristallin, ayant un réseau constitué par des tétraèdres $SiO_4$ et $AlO_4$ réticulés par le déplacement d'atomes d'oxygène et caractérisée par les distances interplanaires et les intensités de ses raies de diffraction des rayons X suivantes:

| d (Å) | Intensité Relative | d (Å) | Intensité Relative |
|---|---|---|---|
| 8,90±0,18 | F | 3,93±0,08 | F |
| 8,04±0,16 | TI | 3,69±0,08 | I |
| 7,34±0,15 | F | 3,35±0,07 | M |
| 7,01±0,14 | TI | 3,32±0,07 | TI |
| 6,42±0,13 | S | 3,25±0,07 | TI |
| 6,06±0,12 | M | 3,15±0,06 | P |
| 5,75±0,12 | M | 3,11±0,06 | TI |
| 5,64±0,12 | M | 3,03±0,06 | I |
| 5,27±0,11 | M | 2,91±0,06 | F |
| 5,12±0,11 | F | 2,81±0,06 | M |
| 5,00±0,10 | M | 2,72±0,06 | M |
| 4,61±0.09 | TI | 2,64±0,05 | M |
| 4,52±0,09 | M | 2,25±0,05 | F |
| 4,09±0,08 | M | 2,05±0,04 | F |

ladite zéolite possédant la formule

$$M_{2/n}O{:}Al_2O_3{:}6—10\ SiO_2$$

dans laquelle M est un ou plusieurs cations de valence n.

2. Zéolite selon la revendication 1, dans laquelle M représente un métal alcalin et un cation organique azoté.

3. Zéolite selon la revendication 1 ou la revendication 2 qui, sous la forme anhydre, possède la formule

$$(0{,}01—0{,}4)\ R_2O{:}(0{,}9\pm0{,}2)\ M_2O{:}Al_2O_3{:}(6—10)\ SiO_2$$

dans laquelle M est un métal alcalin et R est un cation organique azoté.

4. Zéolite selon la revendication 2 ou la revendication 3, dans laquelle le cation organique azoté contient au moins un groupe alkyle ou aryle ayant de là 7 atomes de carbone.

5. Zéolite selon l'une quelconque des revendications 2 à 4 dans laquelle le cation organique azoté est le [cation] tétraéthylammonium.

6. Zéolite selon l'une quelconque des revendications 1 à 5 dont les cations initiaux ont été au moins partiellement éliminés par traitement thermique et/ou échange d'ions.

7. Zéolite selon la revendication 6, qui contient des cations hydrogène, précurseurs d'hydrogène et/ou des cations [de métaux] des terres rares.

8. Procédé de préparation de la zéolite revendiquée dans la revendication 1 qui consiste à préparer un mélange contenant de la silice, de l'aluminate de sodium, de l'hydroxyde de sodium, du bromure de tétraéthylammonium et de l'eau et ayant la composition exprimée en rapports molaires d'oxydes:

| | |
|---|---|
| $SiO_2/Al_2O_3$ | 5—15 |
| $R^+/R^++M^+$ | 0,4—1,0 |
| $OH^-/SiO_2$ | 0,3—0,7 |
| $H_2O/OH^-$ | 50—300 |

(où $R^+$ est un cation tétraéthylammonium et $M^+$ est un cation de métal alcalin), à maintenir ledit mélange à une température de 80°C à 180°C et une pression de 618,5 kPa (75 psig) à 1480,3 kPa (200 psig) jusqu'à ce que les cristaux de zéolite se forment, et à séparer et recueillir lesdits cristaux.

9. Procédé selon la revendication 8 dans lequel ledit mélange a la composition

| | |
|---|---|
| $SiO_2/Al_2O_3$ | 8—10 |
| $R^+/R^++M^+$ | 0,6—0,8 |
| $OH^-/SiO_2$ | 0,4—0,6 |
| $H_2O/OH^-$ | 60—150 |

et il est maintenu à une température de 121°C à 149°C et une pression dans l'intervalle de 790,8 kPa (100 psig) à 1307,9 kPa (175 psig).

10. Procédé selon la revendication 8 ou la revendication 9 dans lequel $R^+$ est [le cation] dérivé du bromure de tétraéthylammonium et dans lequel $M^+$ est le [cation] sodium.

11. Procéde selon l'une quelconque des revendications 8 à 10 dans lequel ledit mélange est maintenu á une température d'environ 135°C pendant une durée d'environ 96 heures.

12. Utilisation de la zéolite d'aluminosilicate cristallin telle que revendiquée dans l'une quelconque des revendications 1 à 7 comme catalyseur dans la conversion des hydrocarbures.

## Patentansprüche

1. Kristalliner Aluminosilicat-Zeolith mit einer aus $SiO_4$- und $AlO_4$-Tetraedern gebildeten, durch die Teilnahme von Sauerstoffatomen vernetzten Gitterstruktur, gekennzeichnet durch die folgenden interplanaren Abstände und relativen Intensitäten von deren Röntgenstrahlen-Beugungsbanden:

| d (A°) | Relative Intensität | d (A°) | Relative Intensität |
|---|---|---|---|
| 8,90±0,18 | schwach | 3,93±0,08 | schwach |
| 8,04±0,16 | sehr stark | 3,69±0.08 | stark |
| 7,34±0,15 | schwach | 3,35±0,07 | mittel |
| 7,01±0,14 | sehr stark | 3,32±0,07 | sehr stark |
| 6,42±0,13 | stark | 3,25±0,07 | sehr stark |
| 6,06±0,12 | mittel | 3,15±0,06 | mittel |
| 5,75±0,12 | mittel | 3,11±0,06 | sehr stark |
| 5,64±0,12 | mittel | 3,03±0,06 | stark |
| 5,27±0,11 | mittel | 2,91±0,06 | schwach |
| 5,12±0,11 | schwach | 2,81±0,06 | mittel |
| 5,00±0,10 | mittel | 2,72±0,06 | mittel |
| 4,61±0,09 | sehr stark | 2,64±0,05 | mittel |
| 4,52±0,09 | mittel | 2,25±0,05 | schwach |
| 4,09±0,08 | mittel | 2,05±0,04 | schwach |

wobei der Zeolith der Formel entspricht:

$$M_{2/n}O:Al_2O_3:6—10\ SiO_2$$

worin M ein oder mehrere Kationen der Valenz n bedeutet.

2. Zeolith nach Anspruch 1, dadurch gekennzeichnet, daß M die Bedeutung von Alkalimetall und/oder einem stickstoffhaltigen organischen Kation hat.

3. Zeolith nach Anspruch 1 oder 2, dadurch gekennzeichnet, daß die wasserfreie Form der Formel

$$(0,01—0,4)\ R_2O:(0,9\pm0,2\ M_2O:Al_2O_3(6—10)\ SiO_2$$

entspricht, worin M die Bedeutung von Alkalimetall hat und R ein stickstoffhaltiges organisches Kation ist.

4. Zeolith nach Anspruch 2 oder 3, dadurch gekennzeichnet, daß das stickstoffhaltige Kation wenigstens eine Alkyl- oder Arylgruppe mit 1 bis 7 Kohlenstoffatomen enthält.

5. Zeolith nach den Ansprüchen 2 bis 4, dadurch gekennzeichnet, daß das stickstoffhaltige organische Kation Tetraäthylammonium ist.

6. Zeolith nach den Ansprüchen 1 bis 5, dadurch gekennzeichnet, daß dessen ursprüngliche Kationen wenigstens teilweise durch thermische Behandlung und/oder Basenaustausch entfernt worden sind.

7. Zeolith nach Anspruch 6, dadurch gekennzeichnet, daß darin Wasserstoff, Wasserstoff-Vorläufer und/oder Seltenerdmetallkationene enthalten sind.

8. Verfahren zur Herstellung von Zeolithen nach den vorhergehenden Ansprüchen, dadurch gekennzeichnet, daß eine Mischung aus Siliciumdioxid, Natriumaluminat, Natriumhydroxid, Tetraäthylammoniumbromid und Wasser mit einer Zusammensetzung (in Bezug auf die Molverhältnisse von Oxiden) wie folgt gebildet wird:

| | |
|---|---|
| $SiO_2/Al_2O_3$ | 5,0—15 |
| $R^+/R^++M^+$ | 0,4—1,0 |
| $OH^-/SiO_2$ | 0,3—0,7 |
| $H_2O/OH^-$ | 50—300 |

(wobei $R^+$ ein Tetraäthylammoniumkation und $M^+$ ein Alkalimetallkation ist), die Mischung bei einer Temperatur von 80°C bis 180°C und einem Druck von 618,5 kPa (75 psig) bis 1480,3 kPa (200 psig) gehalten wird, bis Kristalle der Zeolithform gebildet werden, und die Kristalle abgetrennt und gewonnen werden.

9. Verfahren nach Anspruch 8, dadurch gekennzeichnet, daß die Mischung die folgende Zusammensetzung hat:

| | |
|---|---|
| $SiO_2/Al_2O_3$ | 8,0—10,0 |
| $R^+/R^++M^+$ | 0,6—0,8 |
| $OH^-/SiO_2$ | 0,4—0,6 |
| $H_2O/OH^-$ | 60—150 |

und bei einer Temperatur innerhalb des Bereiches von 121°C bis 149°C und einem Druck innerhalb des Bereiches von 790,8 kPa (100 psig) bis 1307,9 kPa (175 psig) gehalten wird.

10. Berfahren nach Anspruch 8 oder 9, dadurch gekennzeichnet, daß $R^+$ von Tetraäthylammoniumbromid abgeleitet ist und $M^+$ Natrium ist.

11. Verfahren nach den Ansprüchen 8 bis 10, dadurch gekennzeichnet, daß die Mischung bei einer Temperatur von etwa 135°C während einer Zeit von etwa 96 h gehalten wird.

12. Verwendung des kristallinen Aluminosilicat-Zeolithen gemäß den Ansprüchen 1 bis 7 als Katalysator bei der Umwandlung von Kohlenwasserstoffen.

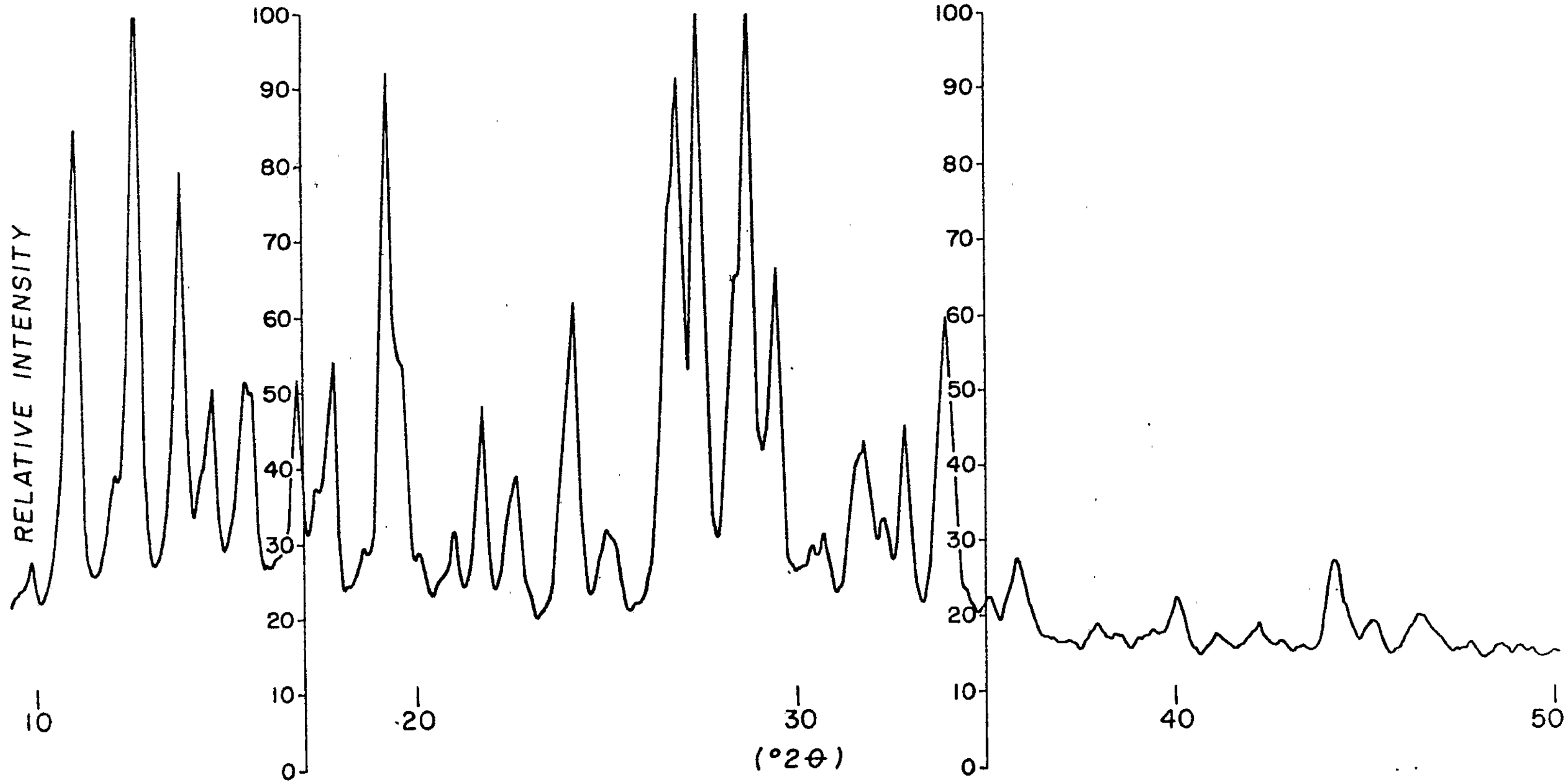


FIG. I

X-RAY DIFFRACTION PATTERN OF ZSM-25
PRODUCT OF EXAMPLE I

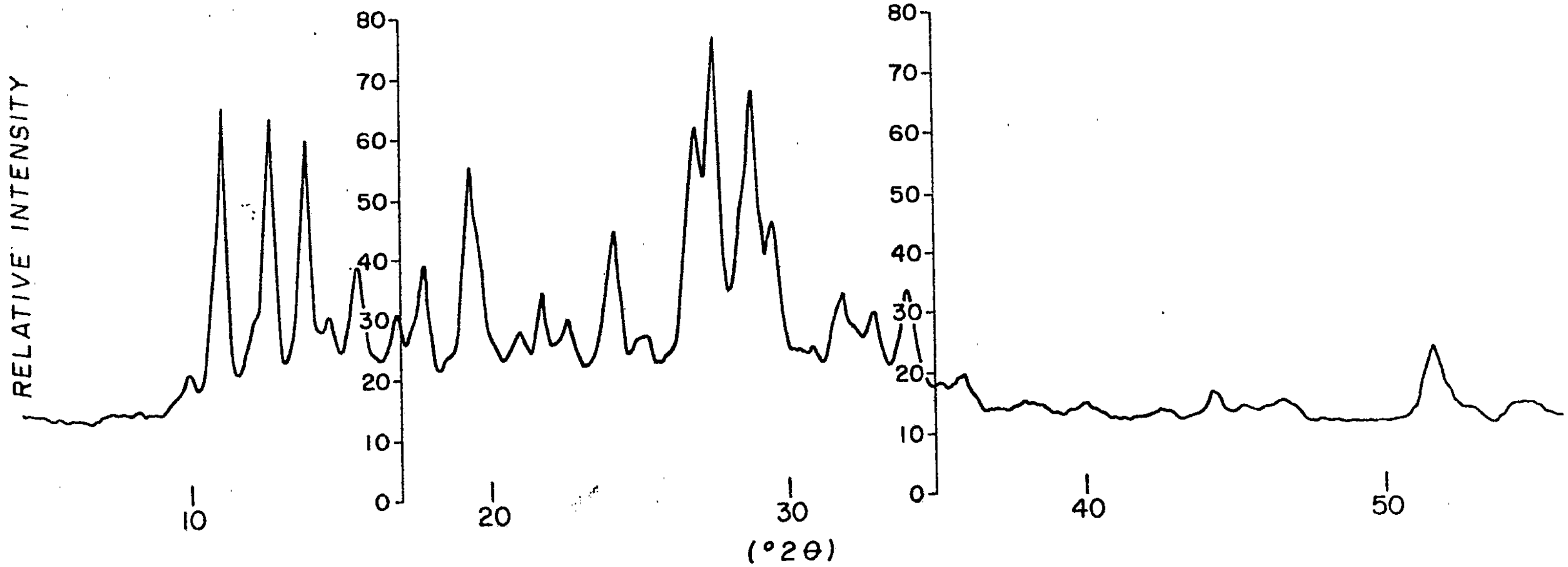


FIG. 2

X-RAY DIFFRACTION PATTERN OF ZSM-25 SAMPLE.
CALCINED FOUR HOURS AT 500°C